# EUROPÄISCHE PATENTSCHRIFT

(11) **EP 1 821 100 B1**
(45) Veröffentlichungstag und Bekanntmachung des Hinweises auf die Patenterteilung: **15.04.2009**
(21) Anmeldenummer: 06101432.0
(22) Anmeldetag: 09.02.2006
(51) Int. Cl.: G01N 33/18, G01N 31/22, B01L 3/00, G01N 21/78, B01L 3/14

(54) **Vorrichtung zur chemischen Analyse von Probeninhaltsstoffen**
A device for the chemical analysis of sample components
Dispositif pour l'analyse chimique des composants d'un échantillon

(43) Veröffentlichungstag der Anmeldung: 22.08.2007
(73) Patentinhaber: Hach Lange GmbH, 14163 Berlin (DE)
(72) Erfinder: LUNDGREEN, Ulrich, 33330 Gütersloh (DE); LENHARD, Markus, 41751 Viersen (DE); FARJAM, Aria, 6291 HD Vaals (NL)
(74) Vertreter: Fitzner, Uwe

(56) Entgegenhaltungen:
- EP-A- 0 663 239
- DE-A1- 10 018 784
- DE-A1- 10 121 999

## Beschreibung

Die vorliegende Erfindung betrifft einen neuartigen Testkit zur chemischen Analyse von gasförmigen oder in die Gasform überführbaren Probeninhaltsstoffen.

In der analytischen Chemie sind solche Methoden von besonderer Bedeutung, bei denen der zu bestimmende Parameter durch Überführung in die Gasform selektiv aus dem Probengemisch abgetrennt wird. Hierbei kann nach Überführung in die Gasphase eine direkte Bestimmung mittels Gaschromatographie, Atomabsorption, oder IR- und Chemolumineszenzspektrometrie durchgeführt werden. Daneben besteht auch die Möglichkeit einer indirekten Bestimmung mit den Methoden der Konduktometrie, der Coulometrie, der Potentiometrie, der Gasvolumetrie, der acidimetrischen Maßanalyse, der manometrischen Messung, der jodometrischen Maßanalyse und der Photometrie.

Die US-5,320,807 beschreibt eine Testvorrichtung, mit der der Zustand und Prozessverlauf in Kompostanlagen überwacht werden kann. Zu diesem Zweck wird eine Probe des Kompostes in ein geschlossenes Gefäß gebracht und dort für einen Zeitraum von einigen Stunden in Ruhe gelassen. Während dieses Zeitraums stellt sich in Gasform des Containers ein Gleichgewicht ein, das durch Austreten von CO₂ und flüchtigen organischen Säuren aus dem Kompost bestimmt wird. Mit einem oder mehreren Nachweisreagenzien, die in dem Gasraum des Containers aufgehängt sind, ist es sodann möglich, die Konzentration von CO₂ oder den organischen flüchtigen Säuren durch die optische Veränderung der Reagenzien nachzuweisen und zu messen. Die gesamte Vorrichtung wird nur für die Untersuchung von Kompostproben eingesetzt. Nachteilig an ihr ist, dass sie keine quantitativen Messungen erlaubt und bei ihrer Befüllung sowohl der Proben- als auch der Nachweisraum zu öffnen sind.

Die US-4,315,890 beschreibt eine Vorrichtung, mit der flüchtige Probenbestandteile, insbesondere aus Körperflüssigkeiten bestimmt werden sollen. Es erfolgt somit keine Erzeugung von Gasen, sondern lediglich die Austreibung vorhandener Gase. Der Nachweis der ausgetriebenen Gase erfolgt in einem geschlossenen Gefäß durch Reaktion mit bzw. Absorption an speziellen, räumlich getrennt angeordneten, gassensitiven Nachweisreagenzien. Die Vorrichtung besteht dabei aus zwei ineinanderschiebbaren Glastuben (nach Art einer Spritze). Auch hierbei ist nachteilig, dass die Vorrichtung während des Befüllens insgesamt offen steht. Eine Verbindung nach außen muss ferner bestehen, um die Gefäßteile ineinander schieben zu können und dabei das verdrängte Volumen auszugleichen.

Aus der WO02/090975 A2 ist ein Verfahren zur fluorimetrischen oder photometrischen Bestimmung von gasförmigen oder in die Gasform überführbaren Stoffen in Proben bekannt. In einer Küvette mit einer oder mehreren ionenpermeablen, gaspermeablen, insbesondere Silicon- und/oder Teflon-Membranen können Aufschlussreaktionen, optionale Reinigungsschritte und die Detektionen durchgeführt werden.

Ferner ist aus der EP1146335B1 ein Testkit für die Analyse von gasförmigen oder in die Gasform überführbaren Inhalfsstoffen einer Probe mit einem Probeaufnahmebehälter für die Aufnahme der Probe über eine Behälteröffnung und mit einem Analysenbehälter für die Aufnahme des zu analysierenden Inhaltsstoffes über eine Behälteröffnung bekannt, wobei der Analysenbehälter ein Indikatorreagenz enthält oder mit einem Indikatorreagenz versehbar und als Messvorlage in einem optischen Messgerät verwendbar ist. Der Testkit ist mit einem Adapter versehen, über den die Behälteröffnungen miteinander verbindbar sind. Der Analysenbehälter enthält eine Druckentlastungsvorrichtung. In dem Behälter ist eine Trennmembran aus einem hydrophoben Material angeordnet. Über die Art des Materials wird in dieser Anmeldung nichts Näheres ausgeführt.

Aus der EP0663239 B1 ist ein Testkit bekannt, welcher zur chemischen Analyse von gasförmigen oder in die Gasform überführbaren Probenstoffen eingesetzt wird. Dieser umfasst zwei separate Behälter von denen einer der Aufnahme der Probe und ein zweiter der Aufnahme der aus der Probe freigesetzten Gase dient. Der zweite Behälter enthält ein gassensitives Reagenz, das durch Kontakt mit dem in dem ersten Behälter erzeugten Gas eine optische Änderung erfährt. Er ist so ausgestaltet, dass er in ein optisches Messgerät als Messvorlage einsetzbar ist. Die Behälter sind über einen Adapter miteinander verbindbar. Der Adapter ist mit einer semipermeablen Membran versehen, die ausschließlich für Gase durchlässig ist. Als Membranmaterial kommen hydrophobe Stoffe in Betracht.

In der WO 00/75653 A2 wird eine Analysevorrichtung beschrieben, die aus zwei ineinanderfügbaren Gefäßen besteht. Hierbei enthält das innere Gefäß den Indikator. Die zu analysierende Probe befindet sich in dem äußeren Gefäß. Beide Gefäße sind nur über den Gasraum miteinander verbunden. Durch Erhitzen werden die flüchtigen Substanzen aus der Probe in die Gasphase befördert und kommen über den Gasraum mit dem Indikator in Kontakt und erzeugen darin eine Änderung. Die Änderung des Indikators wird mittels der Transmission eines Lichtstrahls bestimmt.

Aus der EP 1605260 A2 ist ferner ein Verfahren zur Bestimmung des organisch gebundenen Kohlenstoffs in einer Vorrichtung bekannt, welches wenigstens einen Reaktionsbereich und einen Detektionsbereich aufweist. Hierbei wird die Probe in den Reaktionsbereich der Vorrichtung gegeben, der anorganische Kohlenstoff ausgetrieben, wobei zur Austreibung des durch die Umwandlung des anorganischen Kohlenstoffs entstandenen Kohlendioxids der Reaktionsbereich bewegt wird, die Vorrichtung verschlossen, mittels physikalischer, chemischer, biochemischer oder mikrobiologischer Methoden der organisch gebundene Kohlenstoff und gasförmiges Kohlendioxid umgewandelt, das gasförmige Kohlendioxid in das Detektionsgefäß überführt und anhand der Farbveränderungen des Indikators der Kohlendioxidgehalt mit an sich bekannten Methoden bestimmt.

Verfahren und Vorrichtungen zur Bestimmung des organisch gebundenen Kohlenstoffs sind ferner aus der DE 19616760 A1, WO 99/42824 A1, DE 10018784 C2, DE 10121999 A1, DE 2534620 A1 bekannt.

Um den organisch gebundenen Kohlenstoff zu bestimmen, ist es regelmäßig erforderlich, den anorganischen Kohlenstoff zu entfernen. So wird beispielsweise in der DE 19906151 A1, der DE 19616760 A1, der DE 4307814 A1, der DE 10018784 C2, der DE 1012199 A1, der EP 0663239 B1 und der WO 00/75653 beschrieben, dass die anorganischen Kohlenstoffverbindungen auch durch Ansäuern und anschließendes Austreiben entfernt werden können.

Die beschriebenen, seit einigen Jahren existierenden Testkits weisen mithin folgenden typischen Arbeitsgang bei der Analysendurchführung auf:
1. Der anorganische Kohlenstoff der Probe wird nach Ansäuern in Kohlendioxid umgewandelt und ausgetrieben,
2. die um den anorganischen Kohlenstoff abgereicherte Probe wird in den Reaktionsbereich des Testkits gegeben,
3. ein chemisches Oxidationsmittel wird zugegeben,
4. der Reaktionsbereich wird über den Gasraum mit einer Indikatorlösung verbunden, welche ein für Kohlendioxid empfindliches Farbreagenz enthält,
5. der Reaktionsbereich wird erhitzt, dabei der chemisch gebundene organische Kohlenstoff durch das Oxidationsmittel in Kohlendioxid umgewandelt und dieses Gas in die Indikatorlösung übertragen,
6. der Reaktionsbereich wird abgekühlt,
7. die Farbveränderung der Indikatorlösung durch das übergetriebene Kohlendioxid wird in einem Fotometer als Extinktion gemessen und aus der Extinktion mittels vorliegender Kalibrierdaten der TOC berechnet,
8. die benutzten Reaktionsbereiche mit den verbrauchten Reagenzien werden in die Testkitpackung zurückgegeben und später zur fachgerechten Entsorgung an den Lieferanten zurückgeschickt.

Zusammenfassend ist festzustellen, dass nach dem beschriebenen Stand der Technik eine Trennung von Reaktionsbereich und Analysenbereich vorgesehen ist. In der Regel werden gasförmige Analyten in eine Indikatorflüssigkeit überführt und dort fotometrisch gemessen.

Die Trennung von Reaktionsbereich und Analysenbereich erfolgt mittels Membranen. So werden beispielsweise in der WO 02/090975 A2 Silikonmembranen, in der EP1146335 A2 und der EP0663239 B1 Teflonmembranen eingesetzt. In der WO 00/75653 A2 ist ein gemeinsamer Gasraum für die Trennung der Behälter vorgesehen.

Die DE 100 18 784 A1 beschreibt z. B. ein Verfahren für die Analyse eines gasförmigen oder in die Gasform überführbaren Inhaltsstoffs einer Probe, bei dem die Probe über eine Behälteröffnung in einen Probeaufnahmebehälter eingefüllt und ein zuvor mit dem Indikatorreagenz versehener Analysebehälter mit seiner Behälteröffnung über einen Adapter mit der Behälteröffnung des Probeaufnahmebehälters verbunden wird. Es ist vorgesehen, dass der Adapter mit einer nur für Gase durchlässigen Trennmembran versehen ist, damit ein Flüssigkeitsaustausch zwischen den beiden Behältern vermieden wird. Die Trennmembran kann beispielsweise aus einem hydrophoben Material bestehen.

Gemäß der DE 101 21 999 A1 wird ein Verfahren zur fluorimetrischen oder fotometrischen Bestimmung von gasförmigen oder in die Gasform überführbaren Stoffen in Proben beschrieben. Danach sollen Aufschluss und Detektion in einem Gefäß erfolgen. Hierzu wird das Gefäß durch eine gaspermeable Membran in wenigstens zwei Bereiche unterteilt.

### Die bisher verwendeten Membranen bringen verschiedene Nachteile mit sich:

Teflonmembranen benötigen ein Stützgewebe. Die Herstellung und Handhabung ist daher aufwändig. Es handelt sich um mehrstückige Elemente, welche aus mehreren kombinierten Einzelteilen bestehen.

Die Silikonmembran sind zwar einstückig, jedoch aufwändig in der Handhabung. So ist der Einsatz in die Küvette mit Komplikationen verbunden. D. h. die Montage ist mit erheblichem Aufwand verbunden.

Das in der WO 00/75653 A2 vorgesehene System ist in der Herstellung weniger aufwändig. Jedoch kann es Schwierigkeiten bei der Handhabung geben. Da es sich um ein offenes System handelt, können auch Unsicherheiten in Handhabung und Analyse gegeben sein.

Gegenstand der Erfindung ist ein Testkit zur chemischen Analyse von gasförmigen oder in die Gasform überführbaren Probeninhaltsstoffen.
a) mit wenigstens zwei separaten Bereichen von denen der erste der Aufnahme der Probe und der zweite der Aufnahme der aus der Probe freigesetzten Gase dient, wobei
b) der zweite Bereich ein gassensitives Reagenz enthält, das durch Kontakt mit dem in dem ersten Bereich erzeugten Gas eine Änderung erfährt und
c) die beiden Bereiche durch ein Trennelement voneinander getrennt sind.

Die vorliegende Erfindung hat sich nunmehr die Aufgabe gestellt, an Stelle der nach dem Stand der Technik üblichen hydrophoben Membranen ein Material zur Verfügung zu stellen, welches technologisch weniger aufwendig ist. Es soll ein preiswert herzustellendes Trennelement zur Verfügung gestellt werden.

Die Aufgabe wird dadurch gelöst, dass
d) das Trennelement einen mittleren Porendurchmesser von 0,5 µm bis 1000 µm aufweist und
e) die Dicke des Trennelements 1 mm bis 20 mm beträgt.

Erfindungsgemäß werden demgemäß makroporöse Trennelemente eingesetzt. Deren mittlerer Porendurchmesser liegt in der Regel über 0,5 µm. Bevorzugt sind erfindungsgemäß Porendurchmesser von 0,5 bis 500 µm, besonders bevorzugt von 0,5 bis 100 µm, ganz besonders bevorzugt von 2 bis 50 µm.

In einer erfindungsgemäß bevorzugten Variante weist das Trennelement eine Dicke (Materialstärke) von 1 bis 10 mm, ganz besonders bevorzugt sind 2 bis 5 mm auf. Der Begriff "Dicke" ist im Sinne von Materialstärke zu verstehen. D.h., sofern es sich bei dem Trennelement um eine Scheibe handelt, handelt es sich im Prinzip um die Höhe des Zylinders. Dasselbe gilt, wenn das Trennelement beispielsweise die Form eines Kegels oder Quaders hat. Sofern das Trennelement in Form einer Kugel ausgeführt ist, ist unter dem Begriff "Dicke" bzw. "Materialstärke" der Durchmesser hiervon zu verstehen.

Das beschriebene Trennelement wird im Folgenden als Fritte bezeichnet. Hierunter sind demgemäß Trennelemente zu verstehen:
1. Einstückiges Trennelement,
2. Makroporöse Struktur, wobei der Porendurchmesser mehr als 0,5 µm beträgt,
3. Dicke von mehr als 1 mm, und weniger als 20 mm,
4. es handelt sich um keine Membran. Solche Membranen sind nämlich von einer äußerst geringen Dicke, folienartig, und werden häufig als sog. "dünne Haut" dargestellt bzw. sind diese flexibel bzw. nicht allein tragfähig.

In einer bevorzugten Ausführungsform enthalten oder sind die Trennelemente Sintermaterialien. In einem bevorzugten Fall handelt es sich um einfache Filter aus porösem Sintermaterial. Diese werden in einem einfachen Sinterverfahren aus feinen Pulvern hergestellt. Dabei bestimmt die Korngröße des Pulvers die spätere Porenbreite der Fritte. Bevorzugt werden Korngrößen von 0,5 bis 500 µm, besonders bevorzugt von 2 bis 50 µm.

In einer weiteren Ausführungsform enthalten die Trennelemente Schwämme, Schaumstoffe oder auch hydrophobe oder hydrophile Varianten. Hydrophobe Varianten werden bevorzugt. In Betracht kommen können auch Gewebe, z.B. Textil-, Cellulose-, Filz-, Glasfaser- oder Metallgewebe.

Unter Schäumen oder Schaumstoffen im Sinne der Erfindung sind Gebilde aus gasgefüllten, kugel- oder polyäderförmigen Zellen zu verstehen, welche durch flüssige, halbflüssige, hochviskose oder feste Zellstege begrenzt werden. Die Zellstege, verbunden über sogenannte Knotenpunkte, bilden ein zusammenhängendes Gerüst. Zwischen den Zellstegen spannen sich Schaumlamellen sogenannte geschlossenzellige Schaumlamellen. Werden die Schaumlamellen zerstört oder fließen sie am Ende der Schaumbildung in die Zellstege zurück, erhält man einen offenzelligen Schaum.

Erfindungsgemäß sind demgemäss für das Trennelement offen- oder geschlossenporige Schäume bzw. offen- und geschlossenzellige Schäume, Schaumstoffe oder Schwämme in dem beschriebenen Sinn anwendbar.

Zusammenfassend ist somit festzustellen, dass grundsätzlich alle schwammartigen Gebilde einsetzbar sind, die die beschriebenen Voraussetzungen erfüllen. Demgemäß sind sowohl natürlich vorkommende Schwämme als auch synthetisch hergestellte Produkte für die Zwecke der Erfindung geeignet.

Ein weiteres Beispiel für die erfindungsgemäß einsetzbaren Trennelemente ist das sog. "Controlled Pore Glass"(CPG). Hierunter ist sogenanntes poröses Glas zu verstehen. Derartige Materialien sind als Trägermaterialien für die Gelchromatographie und die Gaschromatographie bekannt. Sie enthalten im Prinzip ein SiO₂-Skelett sowie B₂O₃. Die Gläser können z.B. aus natriumboratreichem Glas hergestellt werden, indem man durch Tempern eine Entmischung herbeiführt und anschließend die Borat-Phase durch Säubern herauslaugt.

Die Verfahren zur Herstellung der genannten Ausführungsformen zum Einsatz in den erfindungsgemäßen Trennelementen sind weitgehend bekannt. Dies gilt beispielsweise für die Sinterverfahren. Ein weiteres Beispiel für die Herstellung poröser Elemente ist das sog. Leaching".

Unter "Leaching" ist die Extraktion eines Stoffes aus festen Gemengen durch geeignete Lösemittel z.B. mit Wasser zu verstehen. Ein Beispiel für die Extraktion ist das Auskochen. Ebenso ist die Laugung mit Hilfe von Bakterien (Bioleaching) möglich. Derartige Verfahren sind in der Hydrometallurgie zur Aufbereitung und zum Aufschluss von Erzen und aus der Erdölgewinnung aus Erdölsanden und Schiefem bekannt.

Die erfindungsgemäßen Fritten können aus verschiedenen Materialien hergestellt werden. Gebräuchlich sind Kunststoff, Metall oder Glas. Es ist überraschend, dass mit Hilfe dieser Fritten die Trennung von Gas und Flüssigkeit in einfacher Weise erreichbar ist. Im Ergebnis führt der Einsatz der beschriebenen Fritten zu einer wesentlichen Kostenersparnis.

Darüber hinaus ist es erfindungsgemäß überraschend, dass auch nicht hydrophobes Ausgangsmaterial (z. B. Metall) eingesetzt werden kann und die so hergestellten Fritten für die Trennung verwendbar sind.

Durch Variation des Ausgangsmaterials können zusätzliche Funktionalitäten in die Fritte integriert werden. So können durch Einlagern zusätzliche Reaktanden z. B. störende Verunreinigungen aus dem Analysegas entfernt werden. Beispielsweise kann ein Chlorabsorber eingelagert werden. Als Absorbermaterial ist beispielsweise Metallpulver verwendbar. Die Verwendung von Metall- oder metallhaltigen Fritten für den TOC-Test hat den Vorteil, dass störendes Chlorgas, welches durch Oxidation der Probe entsteht, mit dem Metall der Fritte reagiert. Dadurch wird die Indikatorlösung vor dem Chlor geschützt. Die Fritte erfüllt somit zwei Aufgaben zeitgleich, nämlich die Abtrennung von CO₂ von der wässrigen Probe, sowie das Zurückhalten von störendem Gas, z. B. Chlorgas. Weitere Einsatzbeispiele für die Verwendung verschiedener Fritten sind analytische Testkits, bei denen ein gasförmiger Analyt gebildet und für den Nachweis verwendet wird (z. B. Tests für Zyanid, organische Säuren, Ammonium, Arsen, Quecksilber, Chlor usw.).

Die besonderen Vorteile des erfindungsgemäßen Trennelements (Fritte) sind darin zu sehen, dass diese preiswert zu produzieren und einfach zu handhaben ist. Auch kann die Modifikation des Materials, z. B. durch Zusatz von Metallpartikeln zu zusätzlichen Funktionalitäten führen. Z. B. kann hierbei die Reaktion des Indikators mit störendem Chlorgas verhindert werden.

Die beschriebenen Fritten können demgemäß entsprechend der gewünschten Funktion in verschiedenen Formen ausgebildet sein. Abgesehen davon können in dem erfindungsgemäßen Testkit auch mehrere Fritten gleichzeitig eingesetzt werden.

Es können auch durch unterschiedliche Fritten verschiedene Funktionalitäten miteinander kombiniert werden. In einer weiteren Ausführungsform kann die Fritte auch einen Schließmechanismus aufweisen. D. h., das System kann gasdicht in Reaktions- und Probeaufnahmebereich getrennt werden, und je nach Bedarf die Fritte für einen Gasdurchtritt geöffnet oder geschlossen werden.

In einer erfindungsgemäßen Variante umfasst der Testkit zwei separate einzelne Behälter, von denen der erste der Aufnahme der Probe und der zweite der Aufnahme der aus der Probe freigesetzten Gase dient. Das gassensitive Reagenz ist hier in dem zweiten Behälter vorhanden. Der zweite Behälter ist zugleich so ausgestaltet, dass er in ein optisches Messgerät als Messvorlage einsetzbar ist, in welcher die optische Änderung des Indikatorreagenzes gemessen werden kann.

Die Behälter des Testkits sind vorzugsweise mittels eines Adapters miteinander verbindbar. Die Koppelung durch den Adapter verbindet die beiden Behälter. Außerdem schließt sie gleichzeitig gasdicht gegen den Außenraum ab, d.h. er ist so ausgestaltet, dass keine Undichtigkeiten im Adapterbereich auftreten. So soll vermieden werden, dass von außen eintretende Gase oder von ihnen heraustretende Gase das Analyseergebnis verfälschen, mithin ein vollständiger Gasübergang ohne Störeinflüsse erfolgt.

Der Adapter kann dabei so ausgestaltet sein, dass er die oben beschriebene erfindungsgemäße Fritte enthält.

Der Testkit bildet in dieser Ausführungsform somit ein verschließbares Behältnis (aus zwei Behältern und dem Adapter gebildet), in dem innerhalb eines örtlich abgegrenzten Bereiches eine Reaktionszone angeordnet ist, die zur Aufnahme einer Probe und zur Gaserzeugung aus dieser Probe dient, und in dem in einem weiteren abgegrenzten Bereich eine Detektionszone mit einem gassensitiven Reagenz angeordnet ist, die zur Detektion der in der Reaktionszone erzeugten Gase dient, wobei die Reaktionszone mit der Detektionszone nur über den Gasraum chemisch verbunden ist.

Die Anwendung des Testkits mit dem Adapter und dem integrierten Trennelement kann dermaßen aussehen, dass zuerst der Behälter mit der Detektionszone, der zwecks Lagerstabilität mit einem geeigneten Verschluss versehen ist, und eine vorkonfektionierte Indikatorlösung oder ein anderes geeignetes Nachweisreagenz enthält, geöffnet wird, und anstelle des Verschlusses der Adapter mit dem integrierten Trennelement aufgeschraubt wird. Ebenso wird der zweite Behälter, der die Reaktionszone enthält, geöffnet. Auch er kann die zur Analyse notwendigen Stoffe in vorkonfektionierter Form enthalten. Anschließend wird die zu analysierende Probe in den Behälter mit der Reaktionszone gegeben, und beide Behälter werden mit Hilfe des Adapters gasdicht gegen den Außenraum miteinander verbunden. Ggf. ist daraufhin der Behälter mit der Reaktionszone geeignet zu behandeln, z.B. durch Erwärmung, um die Erzeugung und Freisetzung der nachzuweisenden Gase zu fördern bzw. den Gastransfer in die Detektionszone zu beschleunigen. Nach der Freisetzung der Gase und ihrer Reaktion mit dem Nachweisreagenz in der Detektionszone werden die so erzeugten Veränderungen mit geeigneten Meßmethoden nachgewiesen.

Für das feste oder flüssige gassensitive Reagenz kommt beispielsweise eine optisch sensitive Festphasendetektionsschicht, vorzugsweise eine Optodenmembran, zur Anwendung, die in der Detektionszone angeordnet wird. Bei Optodenmembranen handelt es sich um filmartige Schichten auf Polymerbasis, die auf chemische Beeinflussung, z.B. durch die zu analysierenden Gase, mit einer Änderung ihres optischen Verhaltens, beispielsweise einer Farbänderung, reagieren. Solche Optodenmembranen, in diesem Fall bestehend aus plastifizierter Ethylcellulose und einem inkorporierten pH-Indikator mit selektiver Ansprache auf Kohlendioxid, wurden z.B. von A. Mills und Mitarbeitern in Anal. Chem.1992, 64, 1383-1389 beschrieben. In ähnlicher Weise reagieren von M. Kuratli und Mitarbeitern (Anal.Chem.1993, 65, 3473-3479) entwickelte Optodenmembranen auf Basis von plastifiziertem PVC und einem inkorporierten lipophilen Benzaldehydderivat mit einer Änderung ihrer UV-Absorption (λ ₘₐₓ = 256 nm), sobald sie mit Schwefeldioxid in Kontakt gebracht werden.

Eine entsprechende Ausgestaltung des Behältnisses vorausgesetzt, kann das gassensitive Reagenz auch eine Flüssigkeit sein, die vorzugsweise einen gelösten Indikator oder ein Farbreagenz enthält. Vorzugsweise kommen wässrige Systeme in Betracht. Voraussetzung für deren Einsetzbarkeit ist, dass eine Oberflächenspannung vorhanden ist, die gewährleistet, dass die Indikatorflüssigkeit bei der praktischen Anwendung des erfindungsgemäßen Testkits nicht durch die Poren mit den o. g. Größen hindurch tritt. Ebenso sind demgemäß auch nichtwässrige Systeme verwendbar, sofern diese die erforderlichen Oberflächenspannungen aufweisen und zugleich nicht durch die erfindungsgemäßen Porengrößen bei sachgerechter Anwendung des Testkits hindurchtreten.

Für eine Integration der erfindungsgemäßen Vorrichtung in ein marktübliches Analysensystem ist diese so ausgestaltet, dass sie in ein optisches Messgerät als Messvorlage eingesetzt werden kann. Dabei kann es sich insbesondere um die Ausgestaltung als Küvette für ein Photometer handeln.

In Verbindung mit allen vorgenannten Varianten des erfindungsgemäßen Testkits kann dieser für spezielle Anwendungsfälle so modifiziert werden, dass er in mindestens einem Teilbereich (Reaktions- oder Detektionszone) in geeigneter Weise räumlich unterteilt ist, so dass in diesem Teilbereich zunächst voneinander separiert gehaltene Proben, Reagenzien oder Phasen erst durch einfache mechanische Manipulationen, wie z. B. Kippen, Umstülpen oder Umschwenken der Vorrichtung, miteinander vermischt oder in Kontakt gebracht werden können, ohne dass hierzu ein Öffnen der Vorrichtung erforderlich ist.

Der erfindungsgemäße Testkit wird vorzugsweise in einem Verfahren zur chemischen Analyse von gasförmigen oder in die Gasform überführbaren Probeninhaltsstoffen in einer Vorrichtung der oben geschilderten Art angewendet. Bei diesem Verfahren wird die zu analysierende Probe in die Reaktionszone des Behältnisses gegeben. Hiernach werden die gasförmigen oder gasförmig austreibbaren Bestandteile der Probe in die Detektionszone überführt, wo sie durch Reaktion mit dem festen oder flüssigen Reagenz dessen Veränderung bewirken, welche mit bekannten Messverfahren ausgewertet wird. Vorzugsweise handelt es sich dabei um optische Veränderungen und Messverfahren.

Für die Erzeugung der gasförmigen Bestandteile aus der Probe können physikalische, chemische, biochemische oder mikrobiologische Methoden angewandt werden. Als chemische Methoden seien vorzugsweise Ansäuern, Alkalisierung, Oxidation, Reduktion und Derivatisierung genannt.

Erfindungsgemäß wird vorzugsweise durch geeignete technische Maßnahmen dafür gesorgt, dass der Differenzdruck zwischen dem Indikatorbereich und dem Reaktionsbereich gering ist.

Die Überführung der gasförmigen Bestandteile aus dem Behälter mit der Reaktionszone in den Behälter mit der Detektionszone kann aber auch durch Erzeugung eines höheren Gasdrucks im ersteren Behälter oder durch Erzeugung eines verminderten Gasdrucks im zweiten Behälter beschleunigt werden, so dass über den gemeinsamem Gasraum ein Druckausgleich und somit ein Gastransport aus der Reaktions- in die Detektionszone erfolgt. Ein höherer Gasdruck kann z. B. durch eine chemische Reaktion, bei welcher ein Trägergas entsteht, erzeugt werden. Ein verminderter Gasdruck kann z. B. durch Zehrung eines Gases (d. h. durch dessen Absorption) in der Detektionszone bewirkt werden. Ebenso ist der Einbau üblicher Druckentlastungsvorrichtungen möglich. Beispiele sind Ventilkonstruktionen. Auch die in der EP1146335 B1 vorgesehene mittels einer Kanüle durchstechbare Membran kann hier Verwendung finden. Die Erzeugung und Überführung der gasförmigen Probenbestandteile kann auch durch Energiezufuhr zur Reaktionszone und/oder durch chemische oder physikalische Reaktionen erfolgen. Als geeignete Mittel der Energiezufuhr kommen u. a. Erwärmung, Bestrahlung, insbesondere mit Ultraviolett oder Mikrowellen, Ultraschallbehandlung oder ein durch die Reaktionszone fließender elektrischer Strom in Frage.

Ferner ist es möglich, durch Einsatz biologischer oder biochemischer Methoden die Umwandlung zu gasförmigen Verbindungen zu erreichen. D. h. durch Einsatz von Enzymen, Mikroorganismen oder pflanzlichen bzw. tierischen Zellen kann ebenfalls eine Reduktion oder Oxidation erreicht werden, um gasförmige Verbindungen zu erzeugen.

Nach Überführung des Analytgases in die Detektionszone kann es ggf. vorteilhaft sein, dass das zu detektierende Gas dort zunächst nur adsorbiert wird, und die optische Veränderung erst nach Zugabe eines weiteren Reagenzes erfolgt. Dies ist z.B. dann sinnvoll, wenn die Temperaturbelastung infolge der zur Überführung der gasförmigen Bestandteile erforderlichen Erhitzung der Reaktionszone für eine oder mehrere der in der Detektionszone wirksamen Indikatorkomponenten zu hoch ist.

Bei speziellen Anwendungen, z. B. wenn nach Vermischung von Probe und Reagenz eine so spontane Gasfreisetzung erfolgt, dass Gasverluste zu erwarten wären, falls nach Proben- oder Reagenzzugabe die erfindungsgemäße Vorrichtung erst noch mittels Zuschrauben verschlossen werden müsste, gelangt vorteilhafterweise die genannte, partiell abgewandelte Modifikation des erfindungsgemäßen Testkits zum Einsatz. Diese zeichnet sich dadurch aus, dass sie in mindestens einem Teilbereich - z. B. in der Reaktionszone - in geeigneter Weise zur getrennten Aufnahme von Reagenzien bzw. Proben räumlich unterteilt ist, so dass diese Phasen durch mechanische Manipulation (Kippen, Umschwenken, o. ä.) der geschlossenen Vorrichtung miteinander vermischt oder in Kontakt gebracht werden können, um auf diese Weise Gasverluste zu vermeiden.

Gasförmige Bestandteile können insbesondere Kohlendioxid, Halogene, Cyanwasserstoff, Ammoniak, Stickoxide, Schwefelwasserstoff, Schwefeloxide, Sauerstoff, Mercaptane, Phosphin, Arsenwasserstoff, Stibin, Quecksilber (Dampf), Phenole, Halogenwasserstoff, Kohlenwasserstoffe und/oder organische Säuren sein.

Mit dem beschriebenen Verfahren kann sowohl der in der Probe vorhandene anorganisch gebundene Kohlenstoff (Total Inorganic Carbon TIC) als auch der gesamte gebundene Kohlenstoff (Total Carbon TC) bestimmt werden. Dazu muss er in Kohlendioxid überführt werden. Für die Bestimmung des TIC wird diese Überführung durch einfaches Ansäuern erreicht. Für die Bestimmung des TC wird diese Überführung durch Oxidation erreicht.

Eine bevorzugte Auswertungsmethode für die optischen Veränderungen in der Detektionszone besteht in der Photometrie. Des Weiteren ist es im Hinblick auf marktübliche Testkits sinnvoll, zur Durchführung der Analyse benötigte Reagenzien in Form von Fertigtests vorzukonfektionieren und in lagerfähiger Form in den einzelnen verschlossenen Behältern unterzubringen.

Der erfindungsgemäße Testkit kann insbesondere verwandt werden zur chemischen Bestimmung des biologischen Sauerstoffbedarfs (BSB), von gebundenem Kohlenstoff (TC), von anorganisch gebundenem Kohlenstoff (TIC), von organisch gebundenem Kohlenstoff (TOC), von gelöstem organischen Kohlenstoff (DOC), von flüchtigen organisch gebundenem Kohlenstoff (VOC), von ausblasbarem organisch gebundenen Kohlenstoff (POC), von adsorbierbaren organischen Halogenverbindungen (AOX), von gebundenen organischen Halogenverbindungen (TOX), von ausblasbaren organischen Halogenverbindungen (DOX), von gelösten organischen Halogenverbindungen (EOX), von schwerflüchtigen Halogenkohlenwasserstoffen (SHKW), von leichtflüchtigen Halogenkohlenwasserstoffen (LHKW), von gebundenem Stickstoff, von Cyanid, von Schwefel, von Phosphor, von Arsen, von Antimon, von Quecksilber, von Phenolen und von anderen flüchtigen organischen Verbindungen.

Im Folgenden wird die Erfindung anhand der Figuren erläutert.

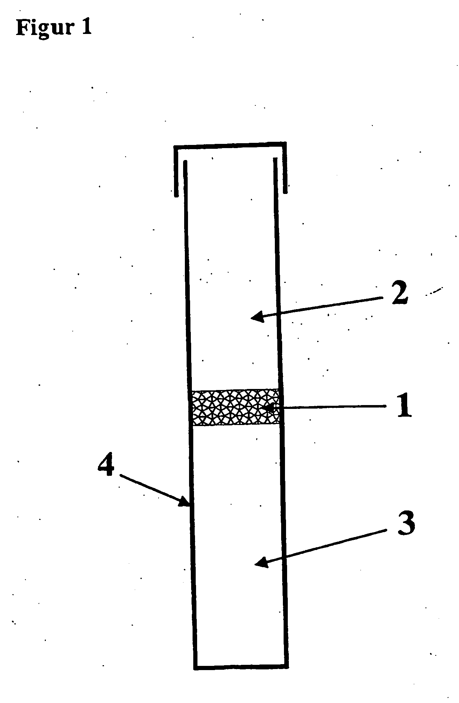

*Figur 1* zeigt eine Vorrichtung, die im Wesentlichen aus einem verschließbaren Behälter besteht.

Diese ist durch eine Fritte 1 in zwei Bereiche, den Reaktionsbereich 2 und den Detektionsbereich 3 getrennt.

Der Reaktionsbereich 2 dient der Aufnahme der Probe und der Gaserzeugung.

Der Detektionsbereich 3 enthält den Indikator, welcher durch Absorption und chemische Reaktion der in Behälter 4 erzeugten Gase eine Änderung (z. B. Farbwechsel) erfährt, die mittels geeigneter bekannter Meßmethoden, wie Photometrie, Fluorimetrie, Luminometrie, Refraktometrie, Reflektometrie und ATR-Photometrie ausgewertet werden kann.

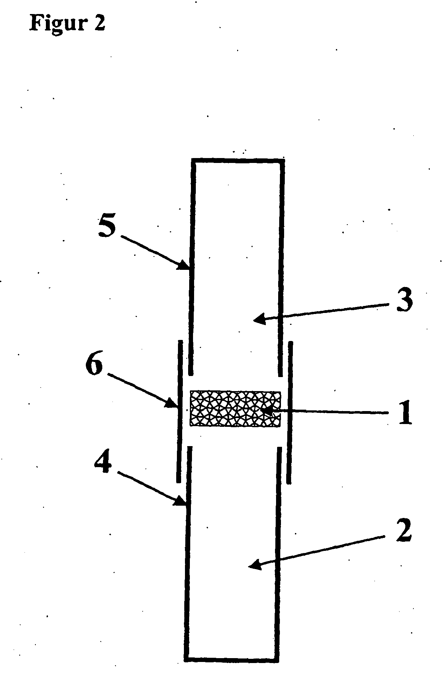

*Figur 2* zeigt eine Ausführung des erfindungsgemäßen Testkits, bei welcher Reaktionszone 2 und Detektionszone 3 des verschließbaren Behältnisses aus zwei separaten Behältern 4,5 bestehen, die über den Adapter 6 miteinander verbunden sind. Der Adapter kann die erfindungsgemäße Fritte 1 oder auch mehrere Fritten enthalten.

Bei dem Testkit enthält der Behälter 4 die Reaktionszone 2, und dient deshalb zur Aufnahme der Probe und zur Gaserzeugung aus derselben. Der Behälter 5 enthält die Detektionszone 3.

## Patentansprüche

1. Testkit zur chemischen Analyse von gasförmigen oder in die Gasform überführbaren Probeninhaltsstoffen,
a) mit wenigstens zwei separaten Bereichen (2,3), von denen der erste (2) der Aufnahme der Probe und der zweite (3) der Aufnahme der aus der Probe freigesetzten Gase dient,
wobei
b) der zweite Bereich (3) ein gassensitives Reagenz enthält, das durch Kontakt mit dem in dem ersten Bereich (2) erzeugten Gas eine Änderung erfährt,
c) die beiden Bereiche (2,3) durch ein Trennelement (1) voneinander getrennt sind,
**dadurch gekennzeichnet, daß** das Trennelement (1) einen mittleren Porendurchmesser von 0,5 bis 500µm aufweist, und
d) die Dicke des Trennelements (1) 1 mm bis 20 mm beträgt.

2. Testkit nach Anspruch 1,
**dadurch gekennzeichnet, dass** das Trennelement (1) Sintermaterialien enthält oder hieraus besteht.

3. Testkit nach Anspruch 2,
**dadurch gekennzeichnet, dass** das Trennelement (1) poröse Sintermaterialien enthält oder hieraus besteht.

4. Testkit nach einem der vorhergehenden Ansprüche,
**dadurch gekennzeichnet, dass** das Trennelement (1) aus Kunststoff, Metall, Glas, Keramik, Aktivkohle, Cellulose oder eines Gemisches einer oder mehrerer dieser Materialien besteht.

5. Testkit nach einem der vorhergehenden Ansprüche
**dadurch gekennzeichnet, dass** das Trennelement (1) Bestandteile enthält, welche mit der Probe, dem Reagenz und/oder dem Gas reagieren.

6. Testkit nach Anspruch 4,
**dadurch gekennzeichnet, dass** das Trennelement (1) als Bestandteile Metallpartikel enthält.

7. Testkit nach einem der vorhergehenden Ansprüche
**dadurch gekennzeichnet, dass** das Trennelement (1) aus offen- oder geschlossenporigen Schaumstoff oder Schwamm besteht.

8. Testkit nach einem der vorhergehenden Ansprüche
**dadurch gekennzeichnet, dass** das Trennelement (1) als Scheibe, Kegel, Quader, Kugel ausgeführt ist.

9. Testkit nach einem der vorgehenden Ansprüche
**dadurch gekennzeichnet, dass** das Trennelement (1) aus Material besteht, welches mit den durchtretenden Gasen reagiert, um Verunreinigungen des Gases zu beseitigen.

10. Testkit nach einem der vorhergehenden Ansprüche
**dadurch gekennzeichnet, dass** die Bereiche (2,3) separate einzelne Behälter (4,5) sind.

11. Testkit nach einem der vorhergehenden Ansprüche
**dadurch gekennzeichnet, dass** die Behälter (4,5) über einen Adapter (6) miteinander verbindbar sind.

12. Testkit nach Anspruch 11
**dadurch gekennzeichnet, dass** in dem Adapter (6) ein Trennelement (1) gemäß einem der Ansprüche 1 bis 7 angeordnet ist.

13. Verfahren zur Herstellung eines Testkists nach einem der Ansprüche 1 - 11
**dadurch gekennzeichnet, dass** das Trennelement (1) durch Sintern aus Kunststoff, Metall, Glas, Keramik, Aktivkohle oder eines Gemisches eines oder mehrerer dieser Materialien oder weiterer Materialien hergestellt wird.

14. Verfahren nach Anspruch 12,
**dadurch gekennzeichnet, dass** das Trennelement (1) durch Leaching hergestellt wird.

15. Verwendung des Testkits nach einem der Ansprüche 1 bis 13
zur chemischen Bestimmung des biologischen Sauerstoffbedarfs (BSB), von gebundenem Kohlenstoff (TC), von anorganisch gebundenem Kohlenstoff (TIC), von organisch gebundenem Kohlenstoff (TOC), von gelöstem organischen Kohlenstoff (DOC), von flüchtigem organisch gebundenen Kohlenstoff (VOC), von ausblasbarem organisch gebundenen Kohlenstoff (POC), von adsorbierbaren organischen Halogenverbindungen (AOX), von gebundenen organischen Halogenverbindungen (TOX), von ausblasbaren organischen Halogenverbindungen (POX), von gelösten organischen Halogenverbindungen (DOX), von extrahierbaren organischen Halogenver bindungen (EOX), von schwerflüchtigen Halogenkohlenwasserstoffen (SHKW), von leichtflüchtigen Halogenkohlenwasserstoffen (LHKW), von gebundenem Stickstoff, von Cyanid, von Schwefel, von Phosphor, von Arsen, von Antimon, von Quecksilber, von Phenolen und von anderen flüchtigen organischen Verbindungen.

## Claims

1. Test kit for the chemical analysis of sample components which are gaseous or convertible into the gas form,
a) having at least two separate regions (2, 3), of which the first (2) serves for receiving the sample and the second (3) serves for receiving the gases liberated from the sample,
b) the second region (3) containing a gas-sensitive reagent which experiences a change due to contact with the gas generated in the first region (2),
c) the two regions (2, 3) being separated from one another by a separation element (1), **characterized in that** the separation element (1) has a mean pore diameter of 0.5 to 500 µm, and
d) the thickness of the separation element (1) is 1 mm to 20 mm.

2. Test kit according to Claim 1, **characterized in that** the separation element (1) contains sintered materials or consists thereof.

3. Test kit according to Claim 2, **characterized in that** the separation element (1) contains porous sintered materials or consists thereof.

4. Test kit according to one of the preceding claims, **characterized in that** the separation element (1) consists of plastic, metal, glass, ceramic, activated carbon, cellulose or a mixture of one or more of these materials.

5. Test kit according to one of the preceding claims, **characterized in that** the separation element (1) contains components which react with the sample, the reagent and/or the gas.

6. Test kit according to Claim4, **characterized in that** the separation element (1) contains metal particles as components.

7. Test kit according to one of the preceding claims, **characterized in that** the separation element (1) consists of open-pore or closed-pore foamed material or sponge.

8. Test kit according to one of the preceding claims, **characterized in that** the separation element (1) is constructed as a disc, cone, parallelepiped, sphere.

9. Test kit according to one of the preceding claims, **characterized in that** the separation element (1) consists of material which reacts with the gases passing through in order to remove impurities of the gas.

10. Test kit according to one of the preceding claims, **characterized in that** the regions (2, 3) are separate individual vessels (4, 5).

11. Test kit according to one of the preceding claims, **characterized in that** the vessels (4, 5) can be connected to one another via an adapter (6).

12. Test kit according to Claim 11, **characterized in that** a separation element (1) according to one of Claims 1 to 7 is arranged in the adapter (6).

13. Method for production of a test kit according to one of Claims 1-11, **characterized in that** the separation element (1) is produced by sintering from plastic, metal, glass, ceramic, activated carbon or a mixture of one or more of these materials or further materials.

14. Method according to Claim 12, **characterized in that** the separation element (1) is produced by leaching.

15. Use of the test kit according to one of Claims 1 to 13 for the chemical determination of biological oxygen demand (BOD), of bound carbon (TC), of inorganically bound carbon (TIC), of organically bound carbon (TOC), of dissolved organic carbon (DOC), of volatile organically bound carbon (VOC), of particulate organically bound carbon (POC), of adsorbable organic halogen compounds (AOX), of bound organic halogen compounds (TOX), of particulate organic halogen compounds (POX), of dissolved organic halogen compounds (DOX), of extractable organic halogen compounds (EOX), of low-volatility halogenated hydrocarbons (SHKW), of highly volatile halogenated hydrocarbons (LHKW), of bound nitrogen, of cyanide, of sulphur, of phosphorus, of arsenic, of antimony, of mercury, of phenols and of other volatile organic compounds.

## Revendications

1. Nécessaire d'essai pour l'analyse chimique de substances gazeuses ou convertissables en la forme gazeuse, contenues dans un échantillon,
a) comportant au moins deux zones séparées (2,3), dont la première (2) sert à la réception de l'échantillon et la deuxième (3) sert à la réception des gaz libérés par l'échantillon,
b) la deuxième zone (3) contenant un réactif sensible aux gaz, qui subit une modification par contact avec le gaz produit dans la première zone (2),
c) les deux zones (2,3) étant séparées l'une de l'autre par un élément séparateur (1),
**caractérisé en ce que** l'élément séparateur (1) présente une taille moyenne de pore de 0,5 à 500 µm, et
d) l'épaisseur de l'élément séparateur (1) va de 1 mm à 20 mm.

2. Nécessaire d'essai selon la revendication 1, **caractérisé en ce que** l'élément séparateur (1) contient ou consiste en des matériaux frittés.

3. Nécessaire d'essai selon la revendication 2, **caractérisé en ce que** l'élément séparateur (1) contient ou consiste en des matériaux frittés poreux.

4. Nécessaire d'essai selon l'une quelconque des revendications précédentes, **caractérisé en ce que** l'élément séparateur (1) est constitué de matière plastique, métal, verre, céramique, charbon actif, cellulose ou d'un mélange d'un ou plusieurs de ces matériaux.

5. Nécessaire d'essai selon l'une quelconque des revendications précédentes, **caractérisé en ce que** l'élément séparateur (1) contient des composants qui réagissent avec l'échantillon, le réactif et/ou le gaz.

6. Nécessaire d'essai selon la revendication 4, **caractérisé en ce que** l'élément séparateur (1) contient comme composants des particules métalliques.

7. Nécessaire d'essai selon l'une quelconque des revendications précédentes, **caractérisé en ce que** l'élément séparateur (1) est constitué d'une éponge ou d'une mousse à pores ouverts ou fermés.

8. Nécessaire d'essai selon l'une quelconque des revendications précédentes, **caractérisé en ce que** l'élément séparateur (1) est réalisé en forme de disque, de cône, de parallélépipède rectangle, de sphère.

9. Nécessaire d'essai selon l'une quelconque des revendications précédentes, **caractérisé en ce que** l'élément séparateur (1) est constitué d'un matériau qui réagit avec les gaz qui le traversent, pour éliminer des impuretés du gaz.

10. Nécessaire d'essai selon l'une quelconque des revendications précédentes, **caractérisé en ce que** les zones (2,3) sont des récipients individuels (4,5) séparés.

11. Nécessaire d'essai selon l'une quelconque des revendications précédentes, **caractérisé en ce que** les récipients (4,5) peuvent être reliés l'un à l'autre par un élément de raccord (6).

12. Nécessaire d'essai selon la revendication 11, **caractérisé en ce qu'**un élément séparateur (1) selon l'une quelconque des revendications 1 à 7 est disposé dans l'élément de raccord (6).

13. Procédé pour la fabrication d'un nécessaire d'essai selon l'une quelconque des revendications 1 à 11, **caractérisé en ce que** l'élément séparateur (1) est produit par frittage à partir de matière plastique, métal, verre, céramique, charbon actif ou d'un mélange d'un ou plusieurs de ces matériaux ou d'autres matériaux.

14. Procédé selon la revendication 12, **caractérisé en ce que** l'élément séparateur (1) est produit par lixiviation.

15. Utilisation du nécessaire d'essai selon l'une quelconque des revendications 1 à 13, pour la détermination chimique de la demande biologique en oxygène (DBO), du carbone lié (TC), du carbone en liaison inorganique (TIC), du carbone en liaison organique (TOC), du carbone organique dissous (DOC), du carbone lié à des substances organiques volatiles (VOC), du carbone lié à des substances organiques volatilisables (POC), des composés halogénés organiques adsorbables (AOX), des composés halogénés organiques liés (TOX), des composés halogénés organiques volatilisables (POX), des composés halogénés organiques dissous (DOX), des composés halogénés organiques extractibles (EOX), des hydrocarbures halogénés peu volatiles (SHKW), des hydrocarbures halogénés volatils (LHKW), de l'azote lié, du cyanure, du soufre, du phosphore, de l'arsenic, de l'antimoine, du mercure, des phénols et d'autres composés organiques volatils.
